# EUROPEAN PATENT SPECIFICATION

(11) **EP 3 710 117 B1**
(45) Date of publication and mention of the grant of the patent: **11.12.2024**
(21) Application number: 18800968.2
(22) Date of filing: 15.11.2018
(51) Int. Cl.: A61Q 17/04, A61K 8/81

(54) **COMPOSITION COMPRISING A UV-SCREENING AGENT, AN ACRYLIC COPOLYMER AND AN ACRYLAMIDOMETHYLPROPANESULFONIC ACID COPOLYMER**
ZUSAMMENSETZUNG MIT EINEM UV-SCHUTZMITTEL, EINEM ACRYLCOPOLYMER UND EINEM ACRYLAMID-METHYLPROPANSULFONSÄURE-COPOLYMER
COMPOSITION COMPRENANT UN FILTRE UV, UN COPOLYMÈRE ACRYLIQUE ET UN COPOLYMÈRE D'ACIDE ACRYLAMIDOMÉTHYLPROPANESULFONIQUE

(30) Priority: 15.11.2017 FR 1760729
(43) Date of publication of application: 23.09.2020
(73) Proprietor: L'OREAL, 75008 Paris (FR)
(72) Inventor: GUIRAMAND, Carole, 94152 Chevilly La Rue (FR); LORANT, Raluca, 94152 Chevilly La Rue (FR); THEROUIN-KOELY, Sandrine, 94152 Chevilly La Rue (FR); CHIRON, Marie-Lise, 94152 Chevilly La Rue (FR); BOUTELET, Karl, 94152 Chevilly La Rue (FR)
(74) Representative: L'Oreal
(86) International application number: PCT/EP2018/081476
(87) International publication number: WO 2019/096961

(56) References cited:
- EP-A1- 3 235 839
- US-A- 4 128 635
- US-A1- 2004 005 279

## Description

The invention relates to a composition, especially a cosmetic composition and in particular a photoprotective composition, and to a process for treating keratin materials, in particular the skin and its integuments, using said composition.

It is known that light radiation with wavelengths of between 280 and 400 nm makes it possible to brown the human epidermis. However, rays with wavelengths more particularly between 280 and 320 nm, known as UVB rays, cause skin erythema and burns which can be detrimental to the development of a natural tan.

For these reasons, and also for aesthetic reasons, there is constant demand for means for controlling this natural tanning in order to control the colour of the skin; this UVB radiation should thus be screened out.

It is also known that UVA rays, with wavelengths of between 320 and 400 nm, and which cause browning of the skin, are liable to induce adverse changes therein, in particular in the case of sensitive skin or skin that is continually exposed to solar radiation. UVA rays cause in particular a loss in the elasticity of the skin and the appearance of wrinkles, resulting in premature skin ageing.

It is therefore desirable also to screen out UVA radiation.

Many photoprotective compositions have been proposed to date for protecting against the effects induced by UVA and/or UVB rays. These compositions generally contain organic or mineral screening agents, more particularly mixtures of organic liposoluble screening agents and/or water-soluble screening agents, combined with metal oxide pigments such as titanium dioxide or zinc oxide. These inorganic particles make it possible to increase the sun protection, which reduces the amount of organic screening agents and thus improves the cosmeticity of the formulations.

Organic screening agents can bring about degraded sensory properties, in particular more greasiness, more tack and/or more sheen.

Stable compositions which have a high SPF without compromising the cosmetic properties such as the greasy finish, tack and sheen are sought. Acrylate polymers are known in the field of cosmetics, in particular in the documents US2004/0005279, EP3235839, US4128635.

The inventors have found, surprisingly, that compositions comprising UV-screening agents, a particular lipophilic polymer and a particular acrylamido-2-propanesulfonic acid copolymer make it possible to obtain stable compositions which have a high SPF and improved cosmetic properties. In particular after application to the skin, there is no shiny effect, the skin is neither greasy nor tacky.

The invention makes it possible to obtain compositions in various presentation forms (for instance cream or milk), which are stable over time and with respect to temperature, and which are sensorily acceptable.

The composition is considered to be stable when its macroscopic and microscopic appearances, its viscosity and its pH show little change, or even no change at all.

The invention relates to a composition, especially a cosmetic composition, in particular a photoprotective composition, comprising at least:
- one or more copolymers described below,
- one or more UV-screening agents, and
- one or more copolymers of acrylamido-2-methylpropanesulfonic acid and of one or more nonionic monomers.

According to the invention, the composition preferably comprises a physiologically acceptable medium.

According to another of its aspects, the invention relates to a photoprotective cosmetic composition comprising, in a physiologically acceptable medium, a composition according to the invention as defined above.

The photoprotective cosmetic composition according to the invention is particularly suitable for performing a non-therapeutic process for the photoprotection of keratin materials.

The photoprotective cosmetic composition according to the invention has, for example, an SPF of at least 5, or even of at least 10, better still 15, better still at least 30, 45 or 60. The SPF (sunscreen protection factor) is defined in the article A new substrate to measure sunscreen protection factors throughout the ultraviolet spectrum, J. Soc. Cosmet. Chem., 40, 127-133 (May/June 1989).

It is known practice to introduce into cosmetic compositions various active agents that are intended to provide specific treatments to the skin and/or the hair. However, some of these active agents have the drawback of being chemically unstable and/or unstable with respect to light. They thus rapidly lose their activity over time, and this instability runs counter to the desired efficacy. The active agents that are particularly concerned are especially resveratrol, retinol, baicalin and vitamin C.

These active agents are very sensitive to certain environmental parameters, for instance light, oxygen and water. Rapid degradation of these formulated active agents thus follows when they are in contact in particular with one of these parameters. Now, it has been sought for a long time to formulate said active agents in the cosmetic and dermatological fields, in various presentation forms, due to the numerous beneficial properties that they have on the skin and/or the hair.

The need thus remains for compositions, and especially an emulsion, containing a photosensitive and/or oxidation-sensitive hydrophilic active agent and especially resveratrol, which is stable and has good cosmetic properties, and which is compatible with the constraints of industrial implementation of its manufacturing process.

The Applicant has discovered, unexpectedly, that the use of a combination of UV-screening agents, a particular lipophilic polymer and a particular acrylamido-2-propanesulfonic acid copolymer in a composition containing an oxidation-sensitive hydrophilic active agent makes it possible to improve the stability of this active agent and thus its efficacy, and to stabilize the composition containing this active agent.

The bioavailability of these active agents is then improved after application to the skin.

A stable composition according to the invention is consequently a composition that shows little or no change in macroscopic appearance (phase separation, change of aspect colour, etc.) or change in microscopic appearance (recrystallization of the active agents) after storage at temperatures of 25°C, 4°C and 40°C, for 2, 4, 8 or 12 weeks and in which the content of active principle remains stable after at least one month at room temperature and at 40°C.

According to a particular mode, the invention relates to a composition, especially a cosmetic composition, in particular a photoprotective composition, comprising at least:
- one polymer described below,
- one or more UV-screening agents, and
- one or more copolymers of acrylamido-2-methylpropanesulfonic acid and of one or more nonionic monomers, and
- one or more photosensitive active agents.

In the text hereinbelow, and unless otherwise indicated, the limits of a range of values are included within that range, especially in the expressions "between ... and ..." and "ranging from ... to ...".

The terms "AMPS" and "acrylamido-2-methylpropanesulfonic (acid)" are used equivalently in the present patent application.

Moreover, the expressions "one or more" and "greater than or equal to" used in the present description are equivalent to the expressions "at least one" and "at least", respectively.

### POLYMER a)

The composition in accordance with the invention comprises at least one polymer a) comprising monomer units of formulae (A) and (B): 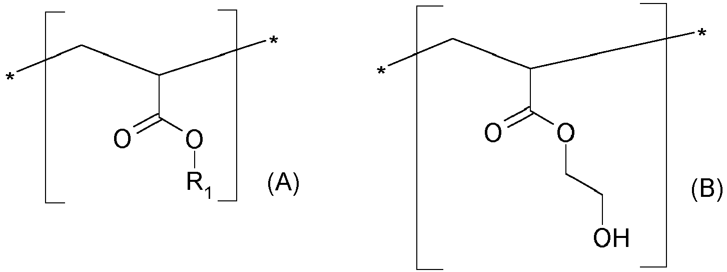 in which:
R1, independently at each instance, is chosen from alkyl and alkylene radicals,
   and
at least 60% by weight of the groups R1 are behenyl radicals, the weight percentage relating to the sum of all the groups R1 present in the polymer,
   and
the weight ratio of the sum of all the hydroxyethyl acrylate units to the sum of all the acrylate units bearing the group R1 ranges from 1:30 to 1:1,
and the sum of the total of units A and B is at least 95% by weight relative to the total weight of the polymer.

Preferably, R1 is constituted of alkyl radicals, preferably of C16-C22 alkyl radicals, and more preferentially of behenyl (C22) radicals.

Preferably, at least 70% by weight of the groups R1 are behenyl radicals, preferentially at least 80% by weight, more preferentially at least 90% by weight.

According to a preferred embodiment, all the groups R1 are behenyl radicals.

Preferably, said weight ratio ranges from 1:15 to 1:1 and preferentially ranges from 1:10 to 1:4.

Advantageously, the polymer units present in the polymer are constituted of the units (A) and (B) previously described.

The polymer has a number-average molecular weight Mn ranging from 2000 to 9000 g/mol, preferably ranging from 5000 to 9000 g/mol. The number-average molecular weight may be measured via the gel permeation chromatography method, for example according to the method described in the example hereinbelow.

Preferably, the polymer has a melting point ranging from 60°C to 69°C and preferentially ranging from 63°C to 67°C. The melting point is measured by differential scanning calorimetry (DSC), for example according to the method described in the example hereinbelow.

The polymer used according to the invention may be prepared by polymerization of a monomer of formula CH2=CH-COO-R1, R1 having the meaning previously described, and of 2-hydroxyethyl acrylate.

The polymerization may be performed according to known methods, such as solution polymerization or emulsion polymerization.

The polymerization is described, for example, in US 2007/0264204.

The lipophilic polymers in accordance with the invention are present in the compositions in concentrations ranging from 0.1% to 10%, even more preferentially from 0.2% to 8% by weight and even more particularly from 0.5% to 5% by weight.

### AMPS COPOLYMER

The compositions according to the invention comprise at least one copolymer of acrylamido-2-methylpropanesulfonic acid or salts thereof and of one or more nonionic monomers.

The AMPS@ copolymers according to the invention may be crosslinked or non-crosslinked.

When the polymers are crosslinked, the crosslinking agents may be chosen from the polyolefinically unsaturated compounds commonly used for crosslinking polymers obtained by radical polymerization.

Examples of crosslinking agents that may be mentioned include divinylbenzene, diallyl ether, dipropylene glycol diallyl ether, polyglycol diallyl ethers, triethylene glycol divinyl ether, hydroquinone diallyl ether, ethylene glycol or tetraethylene glycol di(meth)acrylate, trimethylolpropane triacrylate, methylenebisacrylamide, methylenebismethacrylamide, triallylamine, triallyl cyanurate, diallyl maleate, tetraallylethylenediamine, tetraallyloxyethane, trimethylolpropane diallyl ether, allyl (meth)acrylate, allylic ethers of alcohols of the sugar series, or other allyl or vinyl ethers of polyfunctional alcohols, and also the allylic esters of phosphoric and/or vinylphosphonic acid derivatives, or mixtures of these compounds.

According to a preferred embodiment of the invention, the crosslinking agent is chosen from methylenebisacrylamide, allyl methacrylate and trimethylolpropane triacrylate (TMPTA). The degree of crosslinking generally ranges from 0.01 mol% to 10 mol% and more particularly from 0.2 mol% to 2 mol% relative to the polymer.

The copolymers according to the invention are obtained from AMPS@ and from one or more hydrophilic or hydrophobic ethylenically unsaturated nonionic monomers and, if they are crosslinked, one or more crosslinking agents such as those defined above. When said copolymers include hydrophobic ethylenically unsaturated monomers, these monomers may include one or more fatty chains.

For the purpose of the present invention, the term "fatty chain" means any hydrocarbon-based chain including at least 7 carbon atoms.

The 2-acrylamido-2-methylpropanesulfonic acid monomer of the copolymer contained in the composition in accordance with the invention is in free form or is partially or totally neutralized with a mineral base (sodium hydroxide, potassium hydroxide or aqueous ammonia) or an organic base, such as mono-, di- or triethanolamine, an aminomethylpropanediol, N-methylglucamine, basic amino acids, such as arginine and lysine, and also a mixture of these compounds.

Preferably, the 2-acrylamido-2-methylpropanesulfonic acid monomer according to the invention is partially or totally salified in the form of ammonium or sodium salt.

Preferably, the 2-acrylamido-2-methylpropanesulfonic acid monomer according to the invention is totally salified, preferably in the form of ammonium or sodium salt.

The AMPS@ copolymers according to the invention contain one or more nonionic monomers chosen from water-soluble ethylenically unsaturated monomers, hydrophobic monomers, and mixtures thereof.

Among the nonionic water-soluble monomers, examples that may be mentioned include:
- (meth)acrylamide,
- N-vinylacetamide and N-methyl-N-vinylacetamide,
- N-vinylformamide and N-methyl-N-vinylformamide,
- maleic anhydride,
- vinylamine,
- N-vinyllactams including a cyclic alkyl group containing from 4 to 9 carbon atoms, such as N-vinylpyrrolidone, N-butyrolactam and N-vinylcaprolactam,
- vinyl alcohol of formula CH₂=CHOH,
- the water-soluble vinyl monomers of formula (B) below: 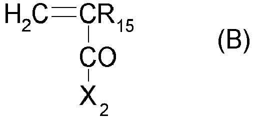
in which:
- R₁₅ is chosen from H, -CH₃, -C₂H₅ and -C₃H₇;
- X₂ is chosen from:
- alkyl oxides of the type -OR₁₆ in which R₁₆ is a linear or branched, saturated or unsaturated hydrocarbon-based radical containing from 1 to 6 carbon atoms, optionally substituted with a halogen (iodine, bromine, chlorine or fluorine) atom; a hydroxyl (-OH) group; ether.

Mention is made, for example, of glycidyl (meth)acrylate, hydroxyethyl (meth)acrylate, and (meth)acrylates of ethylene glycol, of diethylene glycol or of polyalkylene glycol.

Preferably, the water-soluble monomer is chosen from acrylamide, vinylpyrrolidone and hydroxyalkyl (meth)acrylates, more particularly vinylpyrrolidone.

As copolymers of AMPS@ in accordance with the invention with hydrophilic monomers, examples that may be mentioned include:
- copolymers of acrylamido-2-methylpropanesulfonic acid and of vinylpyrrolidone, especially such as the commercial product Aristoflex AVC sold by Clariant,
- crosslinked acrylamide/sodium acrylamido-2-methylpropanesulfonate copolymers, such as that used in the commercial product Sepigel 305^{®} (INCI name: Polyacrylamide/C₁₃-C₁₄ isoparaffin/laureth-7) or that used in the commercial product sold under the name Simulgel 600@ (INCI name: Acrylamide/sodium acryloyldimethyltaurate copolymer/isohexadecane/polysorbate-80^{®}) by the company SEPPIC;
- copolymers of AMPS@ and of hydroxyethyl acrylate, for instance the sodium AMPS^{®}/hydroxyethyl acrylate copolymer, such as that used in the commercial product sold under the name Simulgel NS@ by the company SEPPIC (CTFA name: Hydroxyethyl acrylate/sodium acryloyldimethyltaurate copolymer (and) squalane (and) polysorbate 60).

Among the hydrophobic monomers, examples that may be mentioned include:
- styrene and derivatives thereof, such as 4-butylstyrene, α-methylstyrene and vinyltoluene;
- vinyl acetate of formula CH₂=CH-OCOCH₃;
- vinyl ethers of formula CH₂=CHOR in which R is a linear or branched, saturated or unsaturated hydrocarbon-based radical containing from 1 to 20 and preferably from 1 to 6 carbon atoms;
- acrylonitrile;
- caprolactone;
- vinyl chloride and vinylidene chloride;
- the hydrophobic vinyl monomers of formula (C) below: 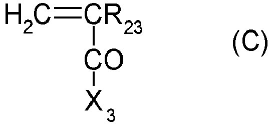
in which:
- R₂₃ is chosen from H, -CH₃, -C₂H₅ and -C₃H₇, preferably H,
- X₃ is chosen from:
- alkyl oxides of the type -OR₂₄ where R₂₄ is a linear or branched, saturated or unsaturated hydrocarbon-based radical containing from 1 to 6 carbon atoms. The hydrocarbon-based radical is preferably an alkyl or an alkenyl. Mention is made, for example, of methyl methacrylate, ethyl methacrylate, n-butyl (meth)acrylate, tert-butyl (meth)acrylate, cyclohexyl acrylate, isobornyl acrylate and 2-ethylhexyl acrylate;
- the vinyl monomers of formula (D) below: 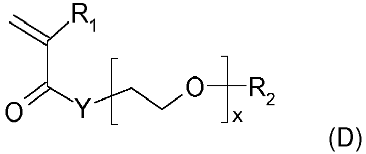 in which R₁ denotes a hydrogen atom or a linear or branched C₁-C₆ alkyl radical, preferably methyl; Y denotes O or NH; R₂ denotes a hydrophobic hydrocarbon-based radical comprising from 8 to 50 carbon atoms, more preferentially from 8 to 22 carbon atoms and even more preferentially from 10 to 18 carbon atoms; x denotes a number ranging from 0 to 100.

According to a particular embodiment of the invention, in formula (D), Y denotes an oxygen atom.

According to a particular embodiment of the invention, in formula (D), the group R₁ represents a methyl.

According to a particular embodiment of the invention, x represents an integer ranging from 3 to 40, and preferably from 5 to 30.

The hydrocarbon-based radical is preferably an alkyl or an alkenyl radical and more particularly an alkyl radical.

The monomers of formula D are preferably chosen from C₈-C₂₂ alkyl (meth)acrylates including from 5 to 40 oxyethylene groups.

The copolymers of AMPS and of a monomer of formula (D) are described especially in patent application FR 2 818 540. The passage in said patent application devoted to the description of these polymers is incorporated into the present patent application.

More particularly, the hydrophobic monomers are chosen from the monomers of formula (D).

The copolymers of AMPS and of hydrophobic monomers are, for example, AMPS/cetearyl methacrylate copolymers ethoxylated with 25 mol of ethylene oxide (80/20), crosslinked with trimethylolpropane triacrylate (TMPTA), sold in particular by Clariant under the name Aristoflex HMS (INCI name: Ammonium acryloyldimethyltaurate/steareth-25 methacrylate crosspolymer) and AMPS/behenyl methacrylate copolymers ethoxylated with 25 mol of ethylene oxide, crosslinked with trimethylolpropane triacrylate (TMPTA) sold by Clariant under the name Aristoflex^{®} HMB (INCI name: Ammonium acryloyldimethyltaurate/beheneth-25 methacrylate crosspolymer).

Use may also be made, as copolymer of AMPS and of a nonionic hydrophobic monomer, of non-crosslinked copolymers of AMPS and of lauryl methacrylate ethoxylated with 7 mol of ethylene oxide, such as Aristoflex^{®} LNC (INCI name: Ammonium acryloyldimethyltaurate/laureth-7 methacrylate copolymer) or non-crosslinked copolymers of AMPS and of stearyl methacrylate ethoxylated with 8 mol of ethylene oxide, such as Aristoflex^{®} SNC (INCI name: Ammonium acryloyldimethyltaurate/steareth-8 methacrylate copolymer).

The AMPS@ copolymers of the invention preferably have a molar mass ranging from 20 000 g/mol to 10 000 000 g/mol, preferably from 80 000 g/mol to 8 000 000 g/mol, and even more preferably from 100 000 g/mol to 7 000 000 g/mol.

As AMPS@ copolymers in accordance with the invention, mention may be made more particularly of:
- copolymers of ammonium acrylamido-2-methylpropanesulfonate and of vinylpyrrolidone, such as the commercial product Aristoflex AVC sold by Clariant,
- AMPS/cetearyl methacrylate copolymers ethoxylated with 25 mol of ethylene oxide, crosslinked with trimethylolpropane triacrylate (TMPTA), sold by Clariant under the name Aristoflex HMS.

According to a more preferred variant of the invention, the copolymers of AMPS and of nonionic monomers according to the invention are copolymers of ammonium acrylamido-2-methylpropanesulfonate and of vinylpyrrolidone.

The AMPS copolymers according to the invention may be present in the composition according to the invention in an amount ranging from 0.1% to 10%, preferably from 0.2% to 5% by weight and more preferentially from 0.5% to 4% by weight, relative to the total weight of the composition.

### UV-screening agents

The composition in accordance with the invention also comprises at least one UV-screening agent (agent for screening out UV radiation from sunlight). The UV-screening agent(s) may be chosen from hydrophilic, lipophilic or insoluble organic UV-screening agents and inorganic UV-screening agents, and mixtures thereof.

The term "UV-screening agent" means a substance that is capable of absorbing at least a portion of the UV radiation emitted by the sun, to protect the skin and/or the lips and/or the hair against the harmful effects of this radiation.

The UV-screening agent is a UV-screening agent normally used in cosmetics. It may be chosen from the positive list contained in Annex VI of (EC) Regulation No. 1223/2009, which specifies the list of UV-screening agents permitted in cosmetics.

According to a particular embodiment, the UV-screening agent(s) are present in the compositions according to the invention in an active material content ranging from 0.1% to 60% by weight and in particular from 5% to 45% by weight, relative to the total weight of the composition.

The water-soluble organic UV-screening agents are especially chosen from the following families:

### Water-soluble screening agents capable of absorbing UV rays from 320 to 400 nm (UVA)

Terephthalylidenedicamphorsulfonic acid manufactured under the name Mexoryl SX by Chimex,
Bis-benzazolyl derivatives as described in EP 669 323 and US 2 463 264, and more particularly the compound disodium phenyldibenzimidazole tetrasulfonate sold under the trade name Neo Heliopan AP by Haarmann & Reimer.

The preferred screening agent is terephthalylidenedicamphorsulfonic acid.

### Water-soluble screening agents capable of absorbing UV rays from 280 to 320 nm (UVB)

*p-Aminobenzoic acid (PABA) derivatives*
PABA,
Glyceryl PABA and
PEG-25 PABA sold under the name Uvinul P25 by BASF,
Phenylbenzimidazolesulfonic acid sold especially under the trade name Eusolex 232 by Merck,
Ferulic acid,
Salicylic acid,
DEA methoxycinnamate,
Benzylidenecamphorsulfonic acid manufactured under the name Mexoryl SL by Chimex,
Camphorbenzalkonium methosulfate manufactured under the name Mexoryl SO by Chimex.

The preferred screening agent is phenylbenzimidazole sulfonic acid.

### Mixed UVA and UVB water-soluble screening agents

*Benzophenone derivatives including at least one sulfonic radical*
Benzophenone-4, sold under the trade name Uvinul MS40 by BASF,
Benzophenone-5, and
Benzophenone-9.

When the absorber is an organic UV-screening agent of sulfonic acid type, it is preferably combined with an amount of an organic base, such as an alkanolamine, so as to make it water-soluble.

The term "alkanolamine" means a C₂-C₁₀ compound comprising at least one primary, secondary or tertiary amine function and at least one alcohol, generally primary alcohol, function.

As suitable alkanolamines, mention may be made of tromethanine and triethanolamine.

The organic screening agents, which are hydrophobic or insoluble in the usual solvents, may be chosen especially from various families of chemical compounds.

### Hydrophobic screening agents capable of absorbing UV rays from 320 to 400 nm (UVA)

*Dibenzoylmethane derivatives*
Butylmethoxydibenzoylmethane sold especially under the trade name Parsol 1789 by DSM Nutritional Products, Inc.,
Isopropyldibenzoylmethane.
*Aminobenzophenones*
n-Hexyl 2-(4-diethylamino-2-hydroxybenzoyl)benzoate, sold under the trade name Uvinul A+ by BASF.
*Anthranilic derivatives*
Menthyl anthranilate sold under the trade name Neo Heliopan MA by Symrise.
*4,4-Diarylbutadiene derivatives*
1,1-Dicarboxy(2,2'-dimethylpropyl)-4,4-diphenylbutadiene.

The preferential screening agents are butylmethoxydibenzoylmethane and n-hexyl 2-(4-diethylamino-2-hydroxybenzoyl)benzoate.

### Hydrophobic screening agents capable of absorbing UV rays from 280 to 320 nm (UVB)

*para-Aminobenzoates*
Ethyl PABA,
Ethyl dihydroxypropyl PABA,
Ethylhexyl dimethyl PABA (Escalol 507 from ISP).
*Salicylic derivatives*
Homosalate sold under the name Eusolex HMS by Rona/EM Industries,
Ethylhexyl salicylate sold under the name Neo Heliopan OS by Symrise,
Dipropylene glycol salicylate sold under the name Dipsal by Scher,
TEA salicylate sold under the name Neo Heliopan TS by Symrise.
*Cinnamates*
Ethylhexyl methoxycinnamate sold especially under the trade name Parsol MCX by DSM Nutritional Products, Inc.,
Isopropyl methoxycinnamate,
Isoamyl methoxycinnamate sold under the trade name Neo Heliopan E 1000 by Symrise,
Diisopropyl methylcinnamate,
Cinoxate,
Glyceryl ethylhexanoate dimethoxycinnamate.
*β,β'-Diphenylacrylate derivatives*
Octocrylene sold especially under the trade name Uvinul N539 by BASF,
Etocrylene sold in particular under the trade name Uvinul N35 by BASF.
*Benzylidenecamphorderivatives*
3-Benzylidenecamphor manufactured under the name Mexoryl SD by Chimex,
Methylbenzylidenecamphor sold under the name Eusolex 6300 by Merck,
Polyacrylamidomethylbenzylidenecamphor manufactured under the name Mexoryl SW by Chimex.
*Triazine derivatives*
Ethylhexyltriazone sold especially under the trade name Uvinul T150 by BASF,
Diethylhexyl butamido triazone, sold under the trade name Uvasorb HEB by Sigma 3V,
2,4,6-Tris(dineopentyl 4'-aminobenzalmalonate)-s-triazine,
2,4,6-Tris(diisobutyl 4'-aminobenzalmalonate)-s-triazine,
2,4-Bis(dineopentyl 4'-aminobenzalmalonate)-6-(n-butyl 4'-am inobenzoate)-s-triazine,
2,4-Bis(n-butyl 4'-aminobenzoate)-6-(aminopropyltrisiloxane)-s-triazine,
the symmetrical triazine screening agents described in patent US 6,225,467, patent application WO 2004/085 412 (see compounds 6 and 9) or the document "Symmetrical Triazine Derivatives" IP.COM Journal, IP.COM Inc West Henrietta, NY, USA (20 September, 2004), especially 2,4,6-tris(biphenyl)-1,3,5-triazines (in particular 2,4,6-tris(biphenyl-4-yl-1,3,5-triazine) and 2,4,6-tris(terphenyl)-1,3,5-triazine, which is also mentioned in the Beiersdorf patent applications WO 06/035000, WO 06/034982, WO 06/034991, WO 06/035007, WO 2006/034992 and WO 2006/034985.

Mention may also be made of 5,6,5',6'-tetraphenyl-3,3'-(1,4-phenylene)bis[1,2,4-triazine] (Phenylene bis-diphenyltriazine).
*Imidazoline derivatives*
Ethylhexyl dimethoxybenzylidene dioxoimidazoline propionate.
*Benzalmalonate derivatives*
Polyorganosiloxanes containing benzalmalonate functions, for instance Polysilicone-15, sold under the trade name Parsol SLX by DSM Nutritional Products, Inc.,
Dineopentyl 4'-methoxybenzalmalonate.
*Merocyanine derivatives*
Octyl 5-N,N-diethylamino-2-phenylsulfonyl-2,4-pentadienoate.

The preferred screening agents are homosalate, ethylhexylsalicylate, octocrylene, ethylhexyl, methoxycinnamate, isoamyl methoxycinnamate, ethylhexyl triazone and diethylhexyl butamido triazone.

The most preferential are ethylhexyl salicylate, octocrylene, ethylhexyl triazone, and ethylhexyl methoxycinnamate.

### Mixed hydrophobic screening agents capable of absorbing both UVA and UVB rays

*Benzophenone derivatives*
Benzophenone-1, sold under the trade name Uvinul 400 by BASF,
Benzophenone-2 sold under the trade name Uvinul D50 by BASF,
Benzophenone-3 or oxybenzone sold under the trade name Uvinul M40 by BASF,
Benzophenone-5,
Benzophenone-6 sold under the trade name Helisorb 11 by Norquay,
Benzophenone-8 sold under the trade name Spectra-Sorb UV-24 by American Cyanamid,
Benzophenone-10,
Benzophenone-11,
Benzophenone-12.
*Phenylbenzotriazole derivatives*

Drometrizole trisiloxane sold under the name Silatrizole by Rhodia Chimie, Methylenebis(benzotriazolyl)tetramethylbutylphenol sold in solid form under the trade name MIXXIM BB/100 by Fairmount Chemical, or in micronized form as an aqueous dispersion under the trade name Tinosorb M by Ciba Specialty Chemicals.

*Bis-resorcinyl triazine derivatives:*
Bis(ethylhexyloxyphenol)methoxyphenyltriazine sold under the trade name Tinosorb S by Ciba Geigy.
*Benzoxazole derivatives*
2,4-Bis[5-1 (dimethylpropyl)benzoxazol-2-yl-(4-phenyl)imino]-6-(2-ethylhexyl)imino-1,3,5-triazine, sold under the name Uvasorb K2A by Sigma 3V.

The preferential screening agents are:
Drometrizole trisiloxane,
Methylene bis-benzotriazolyl tetramethylbutylphenol,
Bis(ethylhexyloxyphenol)methoxyphenyltriazine, and
Benzophenone-3 or Oxybenzone.

The most preferential screening agents are:
Drometrizole trisiloxane, and
Bis(ethylhexyloxyphenol)methoxyphenyltriazine.

Mention may also be made of screening agents of the merocyanine type having the following formula, and also the E/E- or E/Z- geometrical isomeric forms thereof: 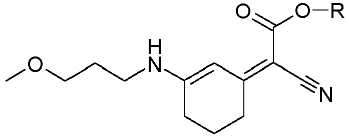 in which:
R is a C₁-C₂₂ alkyl group, a C₂-C₂₂ alkenyl group, a C₂-C₂₂ alkynyl group, a C₃-C₂₂ cycloalkyl group or a C₃-C₂₂ cycloalkenyl group, said groups possibly being interrupted with one or more O.

The merocyanine-type screening agents above may be prepared according to the protocols described in WO 2007/071582, in IP.com Journal (2009), 9(5A), 29-30 IPCOM000182396D under the title "Process for producing 3-amino-2-cyclohexan-1-ylidene compounds" and in US 4 749 643 (column 13, line 66 - column 14, line 57 and the references cited in this regard).

The organic screening agent(s) may be present in the compositions according to the invention in a concentration of between 0.1% and 50% and preferably between 1% and 30% by weight relative to the total weight of the composition.

### Inorganic sunscreens or photoprotective agents

The inorganic photoprotective agents are chosen from coated or uncoated metal oxide pigments (mean size of the primary particles: generally between 5 nm and 100 nm, preferably between 10 nm and 50 nm), for instance titanium oxide (amorphous or crystallized in rutile and/or anatase form), iron oxide, zinc oxide, zirconium oxide or cerium oxide pigments, which are all UV-photoprotective agents that are well known per se.

The pigments may or may not be coated.

The coated pigments are pigments that have undergone one or more surface treatments of chemical, electronic, mechanochemical and/or mechanical nature with compounds as described, for example, in Cosmetics & Toiletries, February 1990, Vol. 105, pages 53-64, such as amino acids, beeswax, fatty acids, fatty alcohols, anionic surfactants, lecithins, sodium, potassium, zinc, iron or aluminium salts of fatty acids, metal alkoxides (of titanium or aluminium), polyethylene, silicones, proteins (collagen, elastin), alkanolamines, silicon oxides, metal oxides or sodium hexametaphosphate.

As is known, silicones are organosilicon polymers or oligomers comprising a linear or cyclic and branched or crosslinked structure, of variable molecular weight, obtained by polymerization and/or polycondensation of suitably functionalized silanes and essentially constituted of a repetition of main units in which the silicon atoms are connected to each other via oxygen atoms (siloxane bond), optionally substituted hydrocarbon-based radicals being connected directly to said silicon atoms via a carbon atom.

The term "silicones" also encompasses the silanes required for their preparation, in particular alkylsilanes.

The silicones used for coating the pigments that are suitable for the present invention are preferably chosen from the group containing alkylsilanes, polydialkylsiloxanes and polyalkylhydrogenosiloxanes. Even more preferentially, the silicones are chosen from the group containing octyltrimethylsilane, polydimethylsiloxanes and polymethylhydrosiloxanes.

Needless to say, before being treated with silicones, the metal oxide pigments may have been treated with other surface agents, in particular with cerium oxide, alumina, silica, aluminium compounds or silicon compounds, or mixtures thereof.

The coated pigments are more particularly titanium oxides that have been coated:
- with silica, such as the product Sunveil from the company Ikeda,
- with silica and iron oxide, such as the product Sunveil F from the company Ikeda,
- with silica and alumina, such as the products Microtitanium Dioxide MT 500 SA and Microtitanium Dioxide MT 100 SA from the company Tayca and Tioveil from the company Tioxide,
- with alumina, such as the products Tipaque TTO-55 (B) and Tipaque TTO-55 (A) from the company Ishihara and UVT 14/4 from the company Kemira,
- with alumina and aluminium stearate, such as the products Microtitanium Dioxide MT 100 T, MT 100 TX, MT 100 Z and MT-01 from the company Tayca, the products Solaveil CT-10 W and Solaveil CT 100 from the company Uniqema and the product Eusolex T-AVO from the company Merck,
- with silica, alumina and alginic acid, such as the product MT-100 AQ from the company Tayca,
- with alumina and aluminium laurate, such as the product Microtitanium Dioxide MT 100 S from the company Tayca,
- with iron oxide and iron stearate, such as the product Microtitanium Dioxide MT 100 F from the company Tayca,
- with zinc oxide and zinc stearate, such as the product BR 351 from the company Tayca,
- with silica and alumina and treated with a silicone, such as the products Microtitanium Dioxide MT 600 SAS, Microtitanium Dioxide MT 500 SAS or Microtitanium Dioxide MT 100 SAS from the company Tayca,
- with silica, alumina and aluminium stearate and treated with a silicone, such as the product STT-30-DS from the company Titan Kogyo,
- with silica and treated with a silicone, such as the product UV-Titan X 195 from the company Kemira,
- with alumina and treated with a silicone, such as the products Tipaque TTO-55 (S) from the company Ishihara or UV Titan M 262 from the company Kemira,
- with triethanolamine, such as the product STT-65-S from the company Titan Kogyo,
- with stearic acid, such as the product Tipaque TTO-55 (C) from the company Ishihara,
- with sodium hexametaphosphate, such as the product Microtitanium Dioxide MT 150 W from the company Tayca;
- TiO₂ treated with octyltrimethylsilane, sold under the trade name T 805 by the company Degussa Silices;
- TiO₂ treated with a polydimethylsiloxane, sold under the trade name 70250 Cardre UF TiO2Sl3 by the company Cardre; and
- anatase/rutile TiO₂ treated with a polydimethylhydrogenosiloxane, sold under the trade name Microtitanium Dioxide USP Grade Hydrophobic by the company Color Techniques.

The uncoated titanium oxide pigments are sold, for example, by the company Tayca under the trade names Microtitanium Dioxide MT 500 B or Microtitanium Dioxide MT 600 B, by the company Degussa under the name P 25, by the company Wackher under the name Transparent titanium oxide PW, by the company Miyoshi Kasei under the name UFTR, by the company Tomen under the name ITS and by the company Tioxide under the name Tioveil AQ.

The uncoated zinc oxide pigments are, for example:
- those sold under the name Z-Cote by the company Sunsmart,
- those sold under the name Nanox by the company Elementis,
- those sold under the name Nanogard WCD 2025 by the company Nanophase Technologies.

The coated zinc oxide pigments are, for example:
- those sold under the name Zinc Oxide CS-5 by the company Toshibi (ZnO coated with polymethylhydrosiloxane),
- those sold under the name Nanogard Zinc Oxide FN by the company Nanophase Technologies (as a 40% dispersion in Finsolv TN, C₁₂-C₁₅ alkyl benzoate),
- those sold under the name Daitopersion ZN-30 and Daitopersion ZN-50 by the company Daito (dispersions in cyclopolymethylsiloxane/oxyethylenated polydimethylsiloxane, containing 30% or 50% of nano zinc oxides coated with silica and polymethylhydrosiloxane),
- those sold under the name NFD Ultrafine ZnO by the company Daikin (ZnO coated with perfluoroalkyl phosphate and copolymer based on perfluoroalkylethyl as a dispersion in cyclopentasiloxane),
- those sold under the name SPD-Z1 by the company Shin-Etsu (ZnO coated with silicone-grafted acrylic polymer, dispersed in cyclodimethylsiloxane),
- those sold under the name Escalol Z100 by the company ISP (alumina-treated ZnO dispersed in an ethylhexyl methoxycinnamate/PVP-hexadecene/methicone copolymer mixture),
- those sold under the name Fuji ZnO-SMS-10 by the company Fuji Pigment (ZnO coated with silica and polymethylsilsesquioxane),
- those sold under the name Nanox Gel TN by the company Elementis (ZnO dispersed at a concentration of 55% in C₁₂-C₁₅ alkyl benzoate with hydroxystearic acid polycondensate).

The uncoated cerium oxide pigments are sold under the name Colloidal Cerium Oxide by the company Rhône-Poulenc.

The uncoated iron oxide pigments are sold, for example, by the company Arnaud under the names Nanogard WCD 2002 (FE 45B), Nanogard Iron FE 45 BL AQ, Nanogard FE 45R AQ and Nanogard WCD 2006 (FE 45R) or by the company Mitsubishi under the name TY-220.

The coated iron oxide pigments are sold, for example, by the company Arnaud under the names Nanogard WCD 2008 (FE 45B FN), Nanogard WCD 2009 (FE 45B 556), Nanogard FE 45 BL 345 and Nanogard FE 45 BL or by the company BASF under the name Transparent Iron Oxide.

Mention may also be made of mixtures of metal oxides, in particular of titanium dioxide and of cerium dioxide, including the equal-weight mixture of titanium dioxide and cerium dioxide coated with silica, sold by the company Ikeda under the name Sunveil A, and also the mixture of titanium dioxide and zinc dioxide coated with alumina, silica and silicone, such as the product M 261 sold by the company Kemira, or coated with alumina, silica and glycerol, such as the product M 211 sold by the company Kemira.

The inorganic screening agent(s) may be present in the compositions according to the invention in a concentration of between 0.1% and 25% and preferably between 0.2% and 10% by weight relative to the total weight of the composition.

### PHOTOSENSITIVE ACTIVE AGENTS

According to a preferred embodiment of the invention, the composition may comprise one or more photosensitive active agents.

The term "photosensitive active agent" thus means an active agent that is chemically and/or physically unstable when exposed to UV radiation. The term "chemical instability" refers especially to degradation of the active principle that may lead, for example, to a colour change in the composition and/or to a decrease in its activity. The term "physical instability" refers especially to crystallization or precipitation of the active agent.

The photosensitive active agents according to the invention may be chosen from:
- vitamins and derivatives thereof,
- retinoic acid and derivatives thereof, in particular retinol and retinyl palmitate, benzene-1,4-bis(3-methylidene-10-camphorsulfonic acid), and n-(C4-C16)acyl-5-salicylic acids such as n-octanoyl-5-salicylic acid,
- polyphenols of natural or synthetic origin, derivatives thereof, and plant extracts comprising same,
- and mixtures thereof.
The vitamins are especially ascorbic acid (vitamin C) and derivatives thereof, especially the phosphate derivatives thereof such as the potassium salt of di-alpha-tocopheryl diascorbyl phosphate (sold by the company Senju Pharmaceutical under the reference Sepivital EPC), magnesium ascorbyl phosphate and sodium ascorbyl phosphate (sold by the company Roche under the reference Stay-C 50), and esters thereof such as ascorbyl acetate, palmitate and propionate; retinol (vitamin A) and derivatives thereof, especially esters thereof such as retinyl acetate, palmitate and propionate; pantothenic acid (vitamin B) and derivatives thereof such as pantolactone, D-panthenol; biotin (vitamin H).

For the purposes of the present invention, the term "polyphenol derivative" refers especially to polyphenol esters, glucosides and phosphates.

Among the polyphenols, mention may be made mainly of phenolic acids and derivatives thereof (chlorogenic acid), and flavonoids, which represent the main subgroup of polyphenols.

Among the flavonoids, mention may be made especially of chalcones, hydroxylated chalcones and derivatives thereof, such as phloretin, neohesperidin, phloridzin and aspalathin; flavanones such as hesperetin and naringin; flavonols such as quercetin and rutin; flavanols such as catechins and EGCG; flavones such as apigenin; and anthocyans.

Mention may also be made of tannins, especially such as ellagic tannins.

The polyphenols may especially derive from plant extracts chosen from green tea, apple, hops, guava or cacao extracts, or from wood, such as chestnut, oak, horse chestnut or hazel, from extracts of Rock Tea or pomegranate extracts, Japanese knotweed (*Fallopia japonica,* also known as *Polygonum cupistadum* or *Reynoutria japonica)* extracts, grape extracts, for instance those from the grapevine species *Vitis vinifera,* blackberry extracts, wine, peanut extracts, and extracts from the following families of plants: *Vitaceae, Myrtaceae, Dipterocarpaceae, Cyperaceae, Gnetaceae, Fabaceae, Pinaceae, Polygonaceae, Moraceae, Fagaceae, Liliaceae,* etc.

The polyphenols are especially baicalin, apigenin, leontopodic acid, ferulic acid, ellagic acid, resveratrol, myricetin and dihydroquercetin.

The preferred photosensitive active agents are chosen from resveratrol, retinol, baicalin and vitamin C, and mixtures thereof, and preferably resveratrol.

The photosensitive and/or oxidation-sensitive active agent(s) may be present in the compositions according to the invention at a concentration ranging from 0.001% to 15% by weight, preferably from 0.01% to 10% by weight and more particularly from 0.1% to 5% by weight relative to the total weight of the composition.

The composition of the invention may comprise an aqueous phase.

The term "aqueous phase" denotes a medium that is liquid at room temperature and atmospheric pressure and which contains a large fraction of water relative to the total weight of the medium. The mass content of water in the aqueous composition is preferably greater than or equal to 10%, advantageously greater than or equal to 30%, preferentially greater than or equal to 40% or even greater than 50% by weight or greater than 80% by weight relative to the total weight of the composition.

The composition may be single-phase or multi-phase.

The composition according to the invention may also contain at least one polar organic solvent, which is preferably physiologically acceptable.

The polar organic solvents are generally water-miscible.

As polar organic solvent, mention may be made of C₁-C₆ monoalcohols such as ethanol or isopropanol; C₁-C₆ polyols such as glycerol, 1,3-propanediol, 1,4-butanediol, 1,5-pentanediol and 1,6-hexanediol; C₁-C₆ alkylene glycols such as ethylene glycol, propylene glycol, dipropylene glycol, butylene glycol, pentylene glycol and hexylene glycol; and mixtures thereof.

The total content of C₁-C₆ alcohols in the composition of the invention is preferably from 0.1% to 10% by weight and preferentially from 1% to 5% by weight of C₁-C₆ alcohols relative to the total weight of the composition.

The total content of C₁-C₆ alkylene glycols in the composition of the invention is preferably from 0.1% to 30% by weight and preferentially from 5% to 25% by weight of C₁-C₆ alkylene glycols relative to the total weight of the composition.

The composition according to the invention may be transparent or translucent, and coloured or uncoloured. The composition according to the invention may contain no pigment or dye. The colouration may correspond to the addition of an additional colouring agent.

The composition according to the invention may include a volatile solvent.

For the purposes of the invention, the term "volatile solvent" means any liquid that is capable of evaporating on contact with keratin materials, at room temperature and atmospheric pressure.

The composition according to the invention may be chosen especially so that the composition contains at least 5%, or even at least 30%, or even at least 40% of volatile solvent.

### Fatty phase

The composition according to the invention may comprise a fatty phase. The fatty phase comprises one or more solid fatty substances, liquid fatty substances such as oils, or pasty fatty substances.

The composition may include an oil, for instance synthetic esters and ethers, linear or branched hydrocarbons of mineral or synthetic origin, fatty alcohols containing from 8 to 26 carbon atoms, partially hydrocarbon-based and/or silicone-based fluoro oils, silicone oils such as volatile or non-volatile polymethylsiloxanes (PDMSs) bearing a linear or cyclic silicone chain, which are liquid or pasty at room temperature, and mixtures thereof, other examples being given hereinbelow.

A composition in accordance with the invention may thus comprise at least one volatile oil.

### Volatile oils

For the purposes of the present invention, the term "volatile oil" means an oil (or non-aqueous medium) that is capable of evaporating on contact with the skin in less than one hour, at room temperature and at atmospheric pressure.

The volatile oil is a volatile cosmetic oil, which is liquid at room temperature, especially having a non-zero vapour pressure at room temperature and atmospheric pressure, in particular having a vapour pressure ranging from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

The volatile hydrocarbon-based oils may be chosen from hydrocarbon-based oils of animal or plant origin containing from 8 to 16 carbon atoms, and especially branched C₈-C₁₆ alkanes (also known as isoparaffins), for instance isododecane (also known as 2,2,4,4,6-pentamethylheptane), isodecane, isohexadecane and, for example, the oils sold under the trade names Isopar^{®} or Permethyl^{®}.

Volatile oils that may also be used include volatile silicones, for instance volatile linear or cyclic silicone oils, especially those with a viscosity ≤ 8 centistokes (8×10⁻⁶ m²/s), and especially containing from 2 to 10 silicon atoms and in particular from 2 to 7 silicon atoms, these silicones optionally including alkyl or alkoxy groups containing from 1 to 10 carbon atoms. As volatile silicone oil that may be used in the invention, mention may be made especially of dimethicones with a viscosity of 5 and 6 cSt, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Volatile fluoro oils such as nonafluoromethoxybutane or perfluoromethylcyclopentane, and mixtures thereof, may also be used.

It is also possible to use a mixture of the oils mentioned above.

### Non-volatile oils

A composition according to the invention may comprise a non-volatile oil.

For the purposes of the present invention, the term "non-volatile oil" means an oil with a vapour pressure of less than 0.13 Pa and especially oils of high molar mass.

The non-volatile oils may be chosen especially from non-volatile hydrocarbon-based oils, which may be fluorinated, and/or silicone oils.

As non-volatile hydrocarbon-based oil that may be suitable for use in the invention, mention may be made especially of:
- hydrocarbon-based oils of animal origin,
- hydrocarbon-based oils of plant origin such as phytostearyl esters, such as phytostearyl oleate, phytostearyl isostearate and lauroyl/octyldodecyl/phytostearyl glutamate, for example sold under the name Eldew PS203 by Ajinomoto, triglycerides constituted of fatty acid esters of glycerol, the fatty acids of which may have chain lengths ranging from C₄ to C₂₄, these chains possibly being linear or branched, and saturated or unsaturated; these oils are especially heptanoic or octanoic triglycerides, wheatgerm oil, sunflower oil, grapeseed oil, sesame oil, corn oil, apricot oil, castor oil, shea oil, avocado oil, olive oil, soybean oil, sweet almond oil, palm oil, rapeseed oil, cotton seed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passion flower oil or musk rose oil; shea butter; or alternatively caprylic/capric acid triglycerides, for instance those sold by the company Stéarinerie Dubois or those sold under the names Miglyol 810^{®}, 812^{®} and 818^{®} by the company Dynamit Nobel,

- hydrocarbon-based oils of mineral or synthetic origin, for instance:
   - synthetic ethers containing from 10 to 40 carbon atoms;
   - linear or branched hydrocarbons of mineral or synthetic origin, such as petroleum jelly, polydecenes, hydrogenated polyisobutene such as Parleam, and squalane, and mixtures thereof, and in particular hydrogenated polyisobutene;
   - synthetic esters, such as the oils of formula R₁COOR₂, in which R₁ represents a linear or branched fatty acid residue including from 1 to 40 carbon atoms and R₂ represents an especially branched hydrocarbon-based chain containing from 1 to 40 carbon atoms, with the proviso that R₁ + R₂ is ≥ 10.

The esters may be chosen especially from especially fatty acid esters, for instance:
- cetostearyl octanoate, isopropyl alcohol esters, such as isopropyl myristate, isopropyl palmitate, ethyl palmitate, 2-ethylhexyl palmitate, isopropyl stearate, isopropyl isostearate, isostearyl isostearate, octyl stearate, hydroxylated esters, for instance isostearyl lactate, octyl hydroxystearate, diisopropyl adipate, heptanoates, and especially isostearyl heptanoate, alcohol or polyalcohol octanoates, decanoates or ricinoleates, for instance propylene glycol dioctanoate, cetyl octanoate, tridecyl octanoate, 2-ethylhexyl 4-diheptanoate, 2-ethylhexyl palmitate, alkyl benzoates, polyethylene glycol diheptanoate, propylene glycol 2-diethylhexanoate, and mixtures thereof, C12-C15 alcohol benzoates, hexyl laurate, neopentanoic acid esters, for instance isodecyl neopentanoate, isotridecyl neopentanoate, isostearyl neopentanoate, octyldodecyl neopentanoate, isononanoic acid esters, for instance isononyl isononanoate, isotridecyl isononanoate, octyl isononanoate, hydroxylated esters, for instance isostearyl lactate, diisostearyl malate and isopropryl lauroyl sarcosinate;
- polyol esters and pentaerythrityl esters, for instance dipentaerythrityl tetrahydroxystearate/tetraisostearate;
- esters of diol dimers and of diacid dimers, such as Lusplan DD-DA5^{®} and Lusplan DD-DA7^{®} sold by the company Nippon Fine Chemical and described in patent application FR 0302809;
- fatty alcohols that are liquid at room temperature, with a branched and/or unsaturated carbon-based chain containing from 12 to 26 carbon atoms, for instance 2-octyldodecanol, isostearyl alcohol, oleyl alcohol, 2-hexyldecanol, 2-butyloctanol and 2-undecylpentadecanol;
- higher fatty acids such as oleic acid, linoleic acid and linolenic acid, and mixtures thereof; and
- dialkyl carbonates, the two alkyl chains possibly being identical or different, such as the dicaprylyl carbonate sold under the name Cetiol CC^{®} by Cognis;
- non-volatile silicone oils, for instance non-volatile polydimethylsiloxanes (PDMSs), polydimethylsiloxanes comprising alkyl or alkoxy groups that are on the side and/or at the ends of a silicone chain, these groups each containing from 2 to 24 carbon atoms, phenyl silicones, for instance phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates, and dimethicones or phenyl trimethicones with a viscosity of less than or equal to 100 cSt, and mixtures thereof;
   - and mixtures thereof.

### Additives

The composition according to the invention may comprise at least one additive chosen from adjuvants that are common in the cosmetic field, such as fillers, colouring agents, hydrophilic or lipophilic gelling agents, water-soluble or liposoluble active agents, preserving agents, moisturizers such as polyols and especially glycerol, sequestrants, antioxidants, solvents, fragrances, odour absorbers, pH adjusters (acids or bases) and mixtures thereof.

The composition may contain at least one active agent which has a supplementary activity in the field of solar protection, such as antioxidants, bleaching agents in the context of anti-pigmentation and depigmentation, and anti-ageing active agents.

The additive(s) may be chosen from those mentioned in the CTFA Cosmetic Ingredient Handbook, 10th Edition Cosmetic and Fragrance Assn, Inc., Washington DC (2004), incorporated herein by reference.

According to a particular embodiment, the composition in accordance with the present invention has a viscosity that may range from 20 mPa.s (ultra-fluid formula) to 100 mPa.s (creamy formula), or even up to 10 Pa.s (thick formulae), the measurements being taken at 25°C with a Rheomat 180 machine with spindle 1, 2, 3 or 4 (depending on the viscosity range) at 200 s⁻¹.

### Presentation forms

The composition according to the invention may be a lotion, a two-phase composition, a cream, a milk, an ointment or a gel, for the skin, the lips, the hair or the nails.

The term "physiologically acceptable medium" means a non-toxic medium that may be applied to keratin materials, in particular the skin, mucous membranes or the integuments.

This medium is adapted to the nature of the support onto which the composition is to be applied, and also to the form in which the composition is intended to be packaged.

The composition may be packaged in any packaging device, in particular made of thermoplastic, or on any support intended for this purpose.

The packaging device may be a bottle optionally with a dropper, a pump-action bottle, an aerosol flask, a tube, a sachet, a jar or a wipe.

### Cosmetic non-therapeutic photoprotection process

The photoprotective cosmetic composition may be applied by hand or using an applicator.

The application may also be performed by spraying or projection using, for example, a piezoelectric or aerograph device or by transfer of a layer of composition previously deposited on an intermediate support.

### EXAMPLES

### Example of preparation of polymer a):

Determination of the molecular weight by gel permeation chromatography (GPC):
The sample is prepared by preparing a solution of the polymer at 10 mg/ml in tetrahydrofuran. The sample is placed in an oven at 54°C for 10 minutes and then in an oscillating shaker for 60 minutes to aid dissolution. After visual inspection, the sample appears to be totally dissolved in the solvent.

The sample prepared was analysed using two polypore 300×7.5 mm columns (manufactured by Agilent Technologies), a Waters 2695 chromatographic system, a tetrahydrofuran mobile phase and detection by refractive index. The sample was filtered through a 0.45 µm nylon filter, before being injected into the liquid chromatograph. The standards used for the calibration are the Easi Vial narrow polystyrene (PS) standards from Agilent Technologies.

Polystyrene standards ranging from 2 520 000 to 162 daltons were used for the calibration.

The system is equipped with a PSS SECcurity 1260 RI detector. The polystyrene calibration curve was used to determine the average molecular weight. The recording of the diagrams and the determination of the various molecular weights were performed by the Win GPC Unichrom 81 program.

Determination of the melting point by differential scanning calorimetry (or DSC):
This method describes the general procedure for determining the melting point of polymers by differential scanning calorimetry. This method is based on the standards ASTM E791 and ASTM D 34182 and the DSC calibration is performed according to standard ASTM E 9672.

### Behenyl acrylate / 2-hydroxyethyl acrylate copolymer (Polymer 1):

In a 4-necked flask equipped with side-blade mixer, an internal thermometer, two funnels, a reflux condenser, and an extension for two other necks, 175 g of behenyl acrylate, 25 g of 2-hydroxyethyl acrylate and 0.4 g of 2,2'-azobis(2-methylbutyronitrile) (Akzo Nobel) were added, over the course of 60 minutes at 80°C, to 40 g of isopropanol, with stirring, after having removed the oxygen from the system by means of a nitrogen flush for 20 minutes. The mixture was stirred at 80°C for 3 hours. The solvent was then removed by vacuum distillation, 1 g of dilauryl peroxide was then added and the reaction was continued for 60 minutes at 110°C. The step was repeated. The mixture was then cooled to 90°C, a stream of demineralized water was added and the mixture was then stirred. The water was removed by vacuum distillation.
Molecular weight: Mn = 7300 g/mol, Mw = 21 000, Mw/Mn = 2.8
Melting point: 65°C

### Examples 1 to 3

For each composition, the viscosity was measured and/or the stability over time was studied at various temperatures and/or the sensory aspect was evaluated during and after its application to the skin.

### Viscosity Measurement

The viscosity is generally measured at 25°C, using a Rheomat RM180@ viscometer equipped with a spindle adapted to the viscosity range and predominantly a No. 3 spindle, the measurement being taken after 10 minutes of rotation of the spindle in the composition (after which time stabilization of the viscosity and of the spin speed of the spindle are observed), at a shear rate of 200 s⁻¹.

### Study of the stability over time at various temperatures

The stability is studied over time by observing the change in the composition with regard to its macroscopic appearance, its microscopic appearance, and the change in viscosity and pH values, at various temperatures such as room temperature (RT), 4°C or 45°C.

### Protocol for evaluating the tack, the shininess and the greasiness

The tacky, shiny and greasy effects of the compositions are evaluated by a panel of sensory experts.

Each composition is applied to the forearm at a dose of 2 mg/cm². The product is spread by circular movements until it has penetrated (approximately 30 seconds). The tacky, shiny and greasy effects are evaluated after 2 minutes of drying, by applying the back of the hand to the treated area, according to a scale ranging from 1 to 15 in which 1 constitutes a reference that is not very tacky or not very shiny or not very greasy, and 15 constitutes a reference that is very tacky or very shiny or very greasy.

In the tables below, SM means starting material and AM means active material.

The following O/W emulsions were prepared:

| | | Example 1 | Example 2 of the invention | Example 3 |
|---|---|---|---|---|
| | | | | |
| C | Disodium salt of ethylenediaminetetraacetic acid, dihydrate | 0.1 | 0.1 | 0.1 |
| C | Triethanolamine | 0.15 | 0.15 | 0.15 |
| D | Triethanolamine | 0.16 | 0.16 | 0.16 |
| C | Preserving agents | qs | qs | qs |
| A | Isononyl isononanoate | 5 | 5 | 5 |
| A | Butylmethoxydibenzoylmethane | 3 | 3 | 3 |
| A | Ethylhexyl salicylate | 5 | 5 | 5 |
| A | Ethylhexyl triazone | 1.5 | 1.5 | 1.5 |
| D | Terephthalylidenedicamphorsulfonic acid | 0.3% AM | 0.3% AM | 0.3% AM |
| A | Octocrylene | 7 | 7 | 7 |
| A | Homosalate | 5 | 5 | 5 |
| A | Bis(ethylhexyloxyphenol)methoxyphenyltriazine | 2 | 2 | 2 |
| D | Carbomer (Synthalen K) | | | 0.38 |
| C | Acrylates copolymer (Carbopol Aqua SF-1 Polymer from Lubrizol) | 0.36% AM | 0.36% AM | 0.36% AM |
| D | Sodium acrylates copolymer (Luvigel EM from BASF) | 0.5% AM | | |
| D | Ammonium acryloyldimethyltaurate/VP copolymer from Clariant | | 0.5% AM | |
| B | Polymer 1 according to the preparation example | 3 | 3 | 3 |
| D | Styrene/acrylates copolymer (Sunsphere Powder) | 1.76% AM | 1.76% AM | 1.76% AM |
| A | Polydimethylsiloxane (viscosity: 5 cSt) | 8 | 8 | 8 |
| D | Denatured absolute ethanol | 10 | 10 | 10 |
| C and D | Deionized water | qs 100% | qs 100% | qs 100% |
| C | Glycerol | 8 | 8 | 8 |

### Preparation method

The starting materials are first weighed out carefully using a balance (precision = 0.01 g).

Compositions 1 to 3 were prepared according to the following procedure:
Heat the fatty phase A with polymer B at 70°C until the polymer has completely melted and dissolved.

In another container, disperse in the aqueous phase C, especially containing, the hydrophilic gelling polymer and neutralize it using the base, then heat the whole phase to 70°C.

Incorporate, with rigorous stirring (rotor/stator type), the oily phase (at 70°C) into the aqueous phase (at 70°C) and cool the emulsion thus obtained to 25°C.

Incorporate phase D with stirring (rotor/stator type) until a smooth homogeneous bulk is obtained.

| | | Ex. 1 | Ex. 2 (invention) | Ex. 3 |
|---|---|---|---|---|
| | Viscosity at t = 24 h | Viscosity: 1600 mPas | Viscosity: 1660 mPas | Viscosity: 3000 mPas |
| | Viscosity at t = 2 months at 4°C/45°C/25°C | Non-compliant Viscosity at 45°C: 2200 mPas | Compliant Viscosity: 1880 mPas | Compliant Viscosity: 3000 mPas |
| | shiny skin finish | 3.6 | 2 | 3 |
| | tacky skin finish | 2.4 | 1 | 1.8 |
| | greasy skin finish | 3.4 | 2.4 | 3 |

The composition according to the invention is stable. It has a sheen, a tacky effect and a greasy finish markedly reduced compared to those of the comparative compositions.

### Example 4:

The following OIW emulsion was prepared:

| | | Example 4 of the invention |
|---|---|---|
| | | |
| C | Disodium salt of ethylenediaminetetraacetic acid, dihydrate | 0.1 |
| C | Triethanolamine | 0.15 |
| D | Triethanolamine | 0.16 |
| C | Preserving agents | qs |
| A | Diisopropyl sebacate | 5 |
| A | Butylmethoxydibenzoylmethane | 3 |
| A | Ethylhexyl salicylate | 5 |
| A | Ethylhexyl triazone | 1.5 |
| D | Terephthalylidenedicamphorsulfonic acid | 0.3% AM |
| A | Octocrylene | 7 |
| A | Homosalate | 5 |
| A | Bis(ethylhexyloxyphenol)methoxyphenyltriazine | 2 |
| C | Acrylates copolymer (Carbopol Aqua SF-1 Polymer from Lubrizol) | 0.36% AM |
| C | Xanthan gum | 0.2 |
| D | Sodium acrylates copolymer (Luvigel EM from BASF) | 0,52%MA |
| D | Ammonium acryloyldimethyltaurate/VP copolymer from Clariant | 0.3% AM |
| B | Polymer 1 according to the preparation example | 3 |
| D | Styrene/acrylates copolymer (Sunsphere Powder) | 1.76% AM |
| A | Polydimethylsiloxane (viscosity: 5 cSt) | 6 |
| A | Vinyl dimethicone/methicone silsesquioxane crosspolymer | 1 |
| A | α,ω-Dihydroxylated polydimethylsiloxane/polydimethylsiloxane mixture 5 cSt (Xiameter PMX-1503 Fluid) | 1 |
| D | Denatured absolute ethanol | 10 |
| C and D | Deionized water | qs 100% |
| C | Glycerol | 8 |

### in vitro SPF

The sun protection factor (SPF) is determined according to the *"in vitro"* method described by B. L. Diffey in J. Soc. Cosmet. Chem. 40, 127-133 (1989). The measurements were taken by means of a UV-2000S spectrophotometer from the company Labsphere. Each composition is applied to a rough PMMA plate, in the form of a uniform and even deposit in a proportion of 1.3 mg/cm².

The composition is stable for 2 months at 4°C, 25°C and 45°C.

It has an *in vitro* SPF of 26.6 ± 0.5 and a PPD UVA index of 10.5 ± 0.2.

The greasy finish and the tack were evaluated by a panel of sensory experts made up of 10 individuals. After application to the skin, the composition is neither tacky (grade of 2.5) nor greasy (grade of 2.5).

### Examples 5 to 7

The following compositions were prepared:

| | Example 5 (invention) | Example 6 | Example 7 | |
|---|---|---|---|---|
| Disodium EDTA | 0.1 | 0.1 | 0.1 | A |
| Lactic acid | 0.275 | 0.275 | 0.275 | H |
| Triethanolamine | 0.37 | 0.37 | 0.37 | A3 |
| Isononyl isononanoate | 2 | 2 | 2 | B |
| Diisopropyl sebacate | 3 | 3 | 3 | B |
| Isopropyl lauryl sarcosinate | 4 | 4 | 4 | B1 |
| Stearyl alcohol | | | 1 | B |
| Elaeis guineensis (palm) oil | 3 | 3 | 3 | B |
| Resveratrol | 0.25 | 0.25 | 0.25 | B1 |
| Butylmethoxydibenzoyl methane | 3 | 3 | 3 | B |
| Ethylhexyl salicylate | 5 | 5 | 5 | B |
| Octocrylene | 7 | 7 | 7 | B |
| Xanthan gum | 0.2 | 0.2 | 0.2 | A1 |
| Methyl methacrylate crosspolymer | 3 | 3 | 3 | E |
| Acrylates copolymer (Aqua SF1) | 0.9 AM | 0.9 AM | 0.9 AM | A2 |
| Poly C10-30 alkyl acrylate (Intelimer IPA 13-1) | | 2 | | B |
| Ammonium acryloyldimethyltaurate/ VP copolymer (Aristoflex AVC) | 0.6 | 0.6 | 0.6 | C |
| Polymer 1 according to the preparation example | 3 | | | B |
| Polydimethylsiloxane (viscosity: 5 cSt) | 2 | 2 | 2 | F |
| Dimethicone (and) dimethicone/vinyl dimethicone crosspolymer | 2 | 2 | 2 | D |
| Vinyl dimethicone/methicone silsesquioxane crosspolymer | 2 | 2 | 2 | F |
| Denatured absolute alcohol | 3 | 3 | 3 | G |
| Deionized water qs | 100% | 100% | 100% | A |
| Glycerol | 8 | 8 | 8 | A |
| Preserving agents | qs | qs | qs | A |
| Glyceryl stearate (and) PEG-100 stearate (Arlacel 165) | | | 0.2 | B |
| Steareth-100 | | | 0.5 | B |
| Niacinamide | 3 | 3 | 3 | A |
| Tocopherol | 0.5 | 0.5 | 0.5 | B |

### Manufacturing process:

Prepare aqueous phase A by heating to 75°C and place under a deflocculator at 450 rpm.

Next, add the xanthan gum and the niacinamide and stir until the composition is homogeneous. Next, add A2, the copolymer and the base.

Heat the resveratrol and the isopropyl lauroyl sarcosinate B1 to 40°C.

Heat phase B to 75°C with magnetic stirring. Just before emulsifying, add B1 to B. Emulsify by adding the hot fatty phase B to the aqueous phase while stirring vigorously with a Moritz blender. Leave for 5 minutes while lowering the temperature (set the nominal value at 25°C). Below 43°C, start stirring with the deflocculator and add phase C (ammonium acηloyldimethyltaurate/VP copolymer) and continue cooling to 25°C.

At 30°C, add the rest of the ingredients D, E and F.

Next, add the alcohol at 25°C and adjust the pH with lactic acid H.

A composition according to the invention was compared with compositions comprising either another polymer (Example 6) or a surfactant system (Example 7).

Composition 5 according to the invention is fine, smooth, homogeneous and glossy. It does not show any crystals under polarized light. It is stable over time (2 months of storage at 45°C).

Compositions 6 and 7 are non-compliant at T0: they show release of oil.

Composition 5 has excellent bioavailability of the resveratrol.

## Claims

1. Composition, especially a cosmetic composition, comprising at least:
- a) one or more polymers comprising monomer units of formulae (A) and (B): 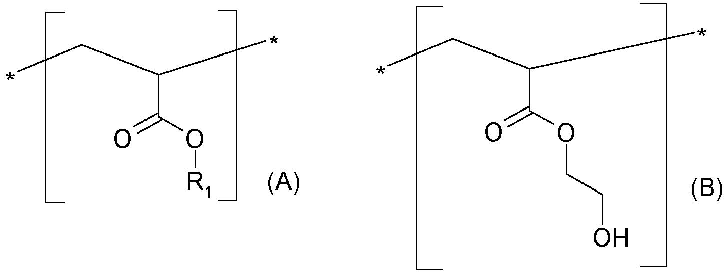 in which:
- R1, independently at each instance, is chosen from alkyl and alkylene radicals,
and
- at least 60% by weight of the groups R1 are behenyl radicals, the weight percentage relating to the sum of all the groups R1 present in the polymer,
and
- the weight ratio of the sum of all the hydroxyethyl acrylate units to the sum of all the acrylate units bearing the group R1 ranges from 1:30 to 1:1;
- and the sum of the total of units A and B is at least 95% by weight relative to the total weight of the polymer,
- the polymer having a number-average molecular weight Mn ranging from 2000 to 9000 g/mol,
- b) one or more UV-screening agents, and
- c) one or more copolymers of acrylamido-2-methylpropanesulfonic acid and of one or more nonionic monomers.

2. Composition according to the preceding claim, in which, in polymer a), R1 is constituted of an alkyl radical, preferably of a C16-C22 alkyl radical, and more preferentially of a behenyl radical.

3. Composition according to either of the preceding claims, in which, in polymer a), at least 70% by weight of the groups R1 are behenyl radicals, preferentially at least 80% by weight, more preferentially at least 90% by weight.

4. Composition according to any one of the preceding claims, in which, in polymer a), all the groups R1 are behenyl radicals.

5. Composition according to any one of the preceding claims, in which, in polymer a), said weight ratio of the sum of all the hydroxyethyl acrylate units to the sum of all the acrylate units bearing the group R1 ranges from 1:15 to 1:1 and preferentially ranges from 1:10 to 1:4.

6. Composition according to any one of the preceding claims, in which polymer a) has a number-average molecular weight Mn ranging from 5000 to 9000 g/mol.

7. Composition according to any one of the preceding claims, in which the lipophilic polymer has a melting point ranging from 60°C to 69°C and preferentially ranging from 63°C to 67°C.

8. Composition according to any one of the preceding claims, in which the content of polymer a) is from 0.01% to 10% by weight, relative to the total weight of said composition.

9. Composition according to any one of Claims 1 to 8, in which the UV-screening agent(s) are chosen from soluble or insoluble organic UV-screening agents, mineral UV-screening agents, and mixtures thereof.

10. Composition according to any one of Claims 1 to 9, in which the content of UV-screening agents is from 0.01% to 60% by weight, relative to the total weight of said composition.

11. Composition according to any one of Claims 1 to 10, in which said copolymers c) comprise one or more nonionic monomers chosen from water-soluble ethylenically unsaturated monomers, hydrophobic monomers, or mixtures thereof.

12. Composition according to any one of the preceding claims, in which said copolymers c) comprise one or more nonionic monomers chosen from the following nonionic water-soluble monomers:
- (meth)acrylamide,
- N-vinylacetamide and N-methyl-N-vinylacetamide,
- N-vinylformamide and N-methyl-N-vinylformamide,
- maleic anhydride,
- vinylamine,
- N-vinyllactams including a cyclic alkyl group containing from 4 to 9 carbon atoms, such as N-vinylpyrrolidone, N-butyrolactam and N-vinylcaprolactam,
- vinyl alcohol of formula CH₂=CHOH,
- water-soluble vinyl monomers of formula (B) below: 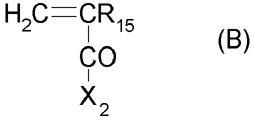
in which:
- R₁₅ is chosen from H, -CH₃, -C₂H₅ and -C₃H₇;
- X₂ is chosen from:
- alkyl oxides of the type -OR₁₆ in which R₁₆ is a linear or branched, saturated or unsaturated hydrocarbon-based radical containing from 1 to 6 carbon atoms, optionally substituted with a halogen (iodine, bromine, chlorine or fluorine) atom; a hydroxyl (-OH) group; ether.

13. Composition according to any one of the preceding claims, in which said copolymers c) comprise one or more nonionic monomers chosen from the following nonionic hydrophobic monomers:
- styrene and derivatives thereof, such as 4-butylstyrene, α-methylstyrene and vinyltoluene;
- vinyl acetate of formula CH₂=CH-OCOCH₃;
- vinyl ethers of formula CH₂=CHOR in which R is a linear or branched, saturated or unsaturated hydrocarbon-based radical containing from 1 to 20 and preferably from 1 to 6 carbon atoms;
- acrylonitrile,
- caprolactone;
- vinyl chloride and vinylidene chloride;
- the hydrophobic vinyl monomers of formula (C) below: 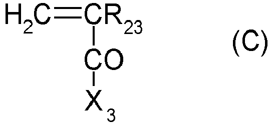
in which:
- R₂₃ is chosen from H, -CH₃, -C₂H₅ and -C₃H₇, preferably H,
- X₃ is chosen from:
- alkyl oxides of the type -OR₂₄ where R₂₄ is a linear or branched, saturated or unsaturated hydrocarbon-based radical containing from 1 to 6 carbon atoms;
- the vinyl monomers of formula (D) below: 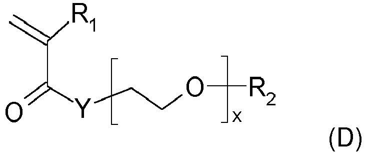
in which R₁ denotes a hydrogen atom or a linear or branched C₁-C₆ alkyl radical, preferably methyl; Y denotes O or NH; R₂ denotes a hydrophobic hydrocarbon-based radical comprising from 8 to 50 carbon atoms, more preferentially from 8 to 22 carbon atoms and even more preferentially from 10 to 18 carbon atoms; x denotes a number ranging from 0 to 100.

14. Composition according to any one of the preceding claims, in which said copolymers c) are chosen from:
- copolymers of ammonium acrylamido-2-methylpropanesulfonate and of vinylpyrrolidone,
- AMPS/cetearyl methacrylate copolymers ethoxylated with 25 mol of ethylene oxide.

15. Composition according to any one of the preceding claims, in which said copolymers c) are present in the composition according to the invention in an amount ranging from 0.1% to 10%, preferably from 0.2% to 8% by weight and more preferentially from 0.5% to 5% by weight, relative to the total weight of the composition.

16. Composition according to any one of the preceding claims, **characterized in that** it also comprises one or more photosensitive active agents.

17. Composition according to the preceding claim, in which the photosensitive active agent(s) are chosen from:
- vitamins and derivatives thereof,
- retinoic acid and derivatives thereof, in particular retinol and retinyl palmitate, benzene-1,4-bis(3-methylidene-10-camphorsulfonic acid), and n-(C4-C16)acyl-5-salicylic acids such as n-octanoyl-5-salicylic acid,
- polyphenols of natural or synthetic origin, derivatives thereof, and plant extracts comprising same,
- and mixtures thereof.

18. Composition according to the preceding claim, in which said photosensitive active agents are chosen from resveratrol, retinol, baicalin and vitamin C, and mixtures thereof, and preferably resveratrol.

19. Composition according to either of Claims 17 and 18, in which the photosensitive active agent(s) are present in a concentration ranging from 0.001% to 15%, preferably from 0.01% to 10% and more particularly from 0.1% to 5% by weight relative to the total weight of the composition.

## Patentansprüche

1. Zusammensetzung, insbesondere kosmetische Zusammensetzung, umfassend mindestens:
- a) ein oder mehrere Polymere, umfassend Monomereinheiten der Formeln (A) und (B): 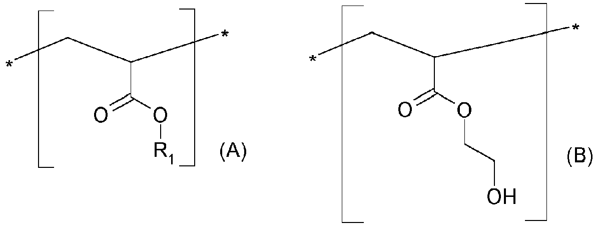 in der:
- R₁ jeweils unabhängig aus Alkyl- und Alkylenresten ausgewählt ist und
- es sich bei mindestens 60 Gew.-% der R₁-Gruppen um Behenylreste handelt, wobei sich der Gewichtsprozentanteil auf die Summe aller in dem Polymer vorliegenden R₁-Gruppen bezieht, und
- das Gewichtsverhältnis der Summe aller Hydroxyethylacrylat-Einheiten zur Summe aller Acrylat-Einheiten, die die R₁-Gruppe tragen, im Bereich von 1:30 bis 1:1 liegt;
- und die Summe der Gesamtheit der Einheiten A und B mindestens 95 Gew.-%, bezogen auf das Gesamtgewicht des Polymers, beträgt,
- wobei das Polymer ein zahlenmittleres Molekulargewicht Mn im Bereich von 2000 bis 9000 g/mol aufweist,
- b) eine oder mehrere UV-Filtersubstanzen und
- c) ein oder mehrere Copolymere von Acrylamido-2-methylpropansulfonsäure und von einem oder mehreren nichtionischen Monomeren.

2. Zusammensetzung nach dem vorhergehenden Anspruch, wobei in dem Polymer a) R₁ aus einem Alkylrest, vorzugsweise einem C₁₆-C₂₂-Alkylrest und weiter bevorzugt einem Behenylrest besteht.

3. Zusammensetzung nach einem der vorhergehenden Ansprüche, wobei es sich in dem Polymer a) bei mindestens 70 Gew.-% der Gruppen R₁ um Behenylreste handelt, vorzugsweise mindestens 80 Gew.-%, weiter bevorzugt mindestens 90 Gew.-%.

4. Zusammensetzung nach einem der vorhergehenden Ansprüche, wobei es sich in dem Polymer a) bei allen der Gruppen R₁ um Behenylreste handelt.

5. Zusammensetzung nach einem der vorhergehenden Ansprüche, wobei in dem Polymer a) das Gewichtsverhältnis der Summe aller Hydroxyethylacrylat-Einheiten zur Summe aller Acrylat-Einheiten, die die R₁-Gruppe tragen, im Bereich von 1:15 bis 1:1, vorzugsweise im Bereich von 1:10 bis 1:4, liegt.

6. Zusammensetzung nach einem der vorhergehenden Ansprüche, wobei das Polymer a) ein zahlenmittleres Molekulargewicht Mn im Bereich von 5000 bis 9000 g/mol aufweist.

7. Zusammensetzung nach einem der vorhergehenden Ansprüche, wobei das lipophile Polymer einen Schmelzpunkt im Bereich von 60 °C bis 69 °C und vorzugsweise im Bereich von 63 °C bis 67 °C aufweist.

8. Zusammensetzung nach einem der vorhergehenden Ansprüche, wobei der Gehalt an Polymer a) 0,01 bis 10 Gew.-%, bezogen auf das Gesamtgewicht der Zusammensetzung, beträgt.

9. Zusammensetzung nach einem der Ansprüche 1 bis 8, wobei die UV-Filtersubstanz bzw. die UV-Filtersubstanzen aus löslichen oder unlöslichen organischen UV-Filtersubstanzen, mineralischen UV-Filtersubstanzen und Mischungen davon ausgewählt ist bzw. sind.

10. Zusammensetzung nach einem der Ansprüche 1 bis 9, wobei der Gehalt an UV-Filtersubstanzen 0,01 bis 60 Gew.-%, bezogen auf das Gesamtgewicht der Zusammensetzung, beträgt.

11. Zusammensetzung nach einem der Ansprüche 1 bis 10, wobei die Copolymere c) ein oder mehrere nichtionische Monomere, die aus wasserlöslichen ethylenisch ungesättigten Monomeren, hydrophoben Monomeren oder Mischungen davon ausgewählt sind, umfassen.

12. Zusammensetzung nach einem der vorhergehenden Ansprüche, wobei die Copolymere c) ein oder mehrere nichtionische Monomere umfassen, die aus den folgenden nichtionischen wasserlöslichen Monomeren ausgewählt sind:
- (Meth)acrylamid,
- N-Vinylacetamid und N-Methyl-N-vinylacetamid,
- N-Vinylformamid und N-Methyl-N-vinylformamid,
- Maleinsäureanhydrid,
- Vinylamin,
- N-Vinyllactamen mit einer cyclischen Alkylgruppe mit 4 bis 9 Kohlenstoffatomen, wie N-Vinylpyrrolidon, N-Butyrolactam und N-Vinylcaprolactam,
- Vinylalkohol der Formel CH₂=CHOH,
- wasserlöslichen Vinylmonomeren der nachstehenden Formel (B): 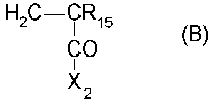 in der:
- R₁₅ aus H, -CH₃, -C₂H₅ und -C₃H₇ ausgewählt ist;
- X₂ ausgewählt ist aus:
- Alkyloxiden des Typs -OR₁₆, wobei R₁₆ für einen linearen oder verzweigten, gesättigten oder ungesättigten kohlenwasserstoffbasierten Rest mit 1 bis 6 Kohlenstoffatomen steht, der gegebenenfalls durch ein Halogenatom (Iod-, Brom-, Chlor- oder Fluoratom); eine Hydroxylgruppe (-OH-Gruppe); Ether substituiert ist.

13. Zusammensetzung nach einem der vorhergehenden Ansprüche, wobei die Copolymere c) ein oder mehrere nichtionische Monomere umfassen, die aus den folgenden nichtionischen hydrophoben Monomeren ausgewählt sind:
- Styrol und Derivaten davon, wie 4-Butylstyrol, α-Methylstyrol und Vinyltoluol;
- Vinylacetat der Formel CH₂=CH-OCOCH₃;
- Vinylethern der Formel CH₂=CHOR, in der R für einen linearen oder verzweigten, gesättigten oder ungesättigten kohlenwasserstoffbasierten Rest mit 1 bis 20 und vorzugsweise 1 bis 6 Kohlenstoffatomen steht;
- Acrylnitril,
- Caprolacton;
- Vinylchlorid und Vinylidenchlorid;
- den hydrophoben Vinylmonomeren der nachstehenden Formel (C): 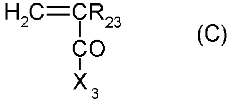 in der:
- R₂₃ aus H, -CH₃, -C₂H₅ und -C₃H₇, vorzugsweise H, ausgewählt ist,
- X₃ ausgewählt ist aus:
- Alkyloxiden des Typs -OR₂₄, wobei R₂₄ für einen linearen oder verzweigten, gesättigten oder ungesättigten kohlenwasserstoffbasierten Rest mit 1 bis 6 Kohlenstoffatomen steht;
- den Vinylmonomeren der nachstehenden Formel (D) : 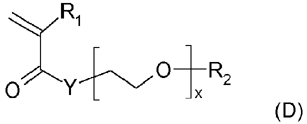 in der R₁ ein Wasserstoffatom oder einen linearen oder verzweigten C₁-C₆-Alkylrest, vorzugsweise Methyl, bedeutet; Y O oder NH bedeutet; R₂ einen hydrophoben kohlenwasserstoffbasierten Rest mit 8 bis 50 Kohlenstoffatomen, weiter bevorzugt 8 bis 22 Kohlenstoffatomen und noch weiter bevorzugt 10 bis 18 Kohlenstoffatomen bedeutet; x eine Zahl im Bereich von 0 bis 100 bedeutet.

14. Zusammensetzung nach einem der vorhergehenden Ansprüche, wobei die Copolymere c) ausgewählt sind aus:
- Copolymeren von Ammoniumacrylamido-2-methylpropansulfonat und von Vinylpyrrolidon,
- AMPS/Cetearylmethacrylat-Copolymeren, die mit 25 mol Ethylenoxid ethoxyliert sind.

15. Zusammensetzung nach einem der vorhergehenden Ansprüche, wobei die Copolymere c) in der erfindungsgemäßen Zusammensetzung in einer Menge im Bereich von 0,1 bis 10 Gew.-%, vorzugsweise 0,2 bis 8 Gew.-% und weiter bevorzugt 0,5 bis 5 Gew.-%, bezogen auf das Gesamtgewicht der Zusammensetzung, vorliegen.

16. Zusammensetzung nach einem der vorhergehenden Ansprüche, **dadurch gekennzeichnet, dass** sie außerdem einen oder mehrere lichtempfindliche Wirkstoffe umfasst.

17. Zusammensetzung nach einem der vorhergehenden Ansprüche, wobei der lichtempfindliche Wirkstoff bzw. die lichtempfindlichen Wirkstoffe aus
- Vitaminen und Derivaten davon,
- Retinsäure und Derivaten davon, insbesondere Retinol und Retinylpalmitat, Benzol-1,4-bis(3-methyliden-10-camphersulfonsäure) und n-(C₄-C₁₆)Acyl-5-salicylsäuren wie n-Octanoyl-5-salicylsäure,
- Polyphenolen natürlicher oder synthetischer Herkunft, Derivaten davon und diese enthaltenden Pflanzenextrakten
- und Mischungen davon
ausgewählt ist bzw. sind.

18. Zusammensetzung nach dem vorhergehenden Anspruch, wobei die lichtempfindlichen Wirkstoffe aus Resveratrol, Retinol, Baicalin und Vitamin C und Mischungen davon und vorzugsweise Resveratrol ausgewählt sind.

19. Zusammensetzung nach Anspruch 17 oder 18, wobei der lichtempfindliche Wirkstoff bzw. die lichtempfindlichen Wirkstoffe in einer Konzentration im Bereich von 0,001 bis 15 Gew.-%, vorzugsweise 0,01 bis 10 Gew.-% und spezieller 0,1 bis 5 Gew.-%, bezogen auf das Gesamtgewicht der Zusammensetzung, vorliegt bzw. vorliegen.

## Revendications

1. Composition, notamment composition cosmétique, comprenant au moins
- a) un ou plusieurs polymères comprenant des motifs monomériques des formules (A) et (B) : 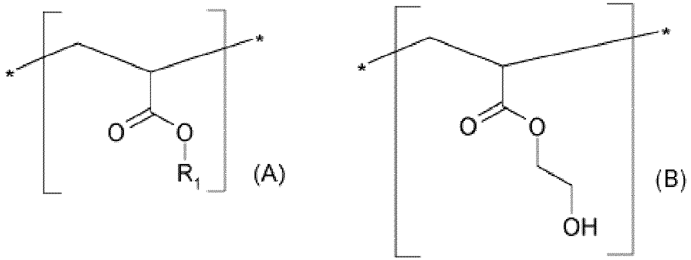 dans lesquelles :
- R1, indépendamment en chaque instance, est choisi parmi des radicaux alkyle et alkylène,
et
- au moins 60 % en poids des groupes R1 sont des radicaux béhényle, le pourcentage en poids faisant référence à la somme de tous les groupes R1 présents dans le polymère, et
- le rapport en poids de la somme de tous les motifs d'acrylate d'hydroxyéthyle sur la somme de tous les motifs d'acrylate portant le groupe R1 se situe dans la plage de 1 : 30 à 1 : 1 ;
- et la somme de la totalité des motifs A et B est d'au moins 95 % en poids par rapport au poids total du polymère,
- le polymère ayant un poids moléculaire moyen en nombre Mn dans la plage de 2 000 à 9 000 g/mole,
- b) un ou plusieurs agents écrans d'UV, et
- c) un ou plusieurs copolymères d'acide acrylamido-2-méthylpropanesulfonique et d'un ou plusieurs monomères non ioniques.

2. Composition selon la revendication précédente, dans laquelle, dans le polymère a), R1 est constitué d'un radical alkyle, préférablement d'un radical alkyle en C16-C22, et plus préférentiellement d'un radical béhényle.

3. Composition selon l'une ou l'autre des revendications précédentes, dans laquelle, dans le polymère a), au moins 70 % en poids des groupes R1 sont des radicaux béhényle, préférentiellement au moins 80 % en poids, plus préférentiellement au moins 90 % en poids.

4. Composition selon l'une quelconque des revendications précédentes, dans laquelle, dans le polymère a), tous les groupes R1 sont des radicaux béhényle.

5. Composition selon l'une quelconque des revendications précédentes, dans laquelle, dans le polymère a), ledit rapport en poids de la somme de tous les motifs d'acrylate d'hydroxyéthyle sur la somme de tous les motifs d'acrylate portant le groupe R1 se situe dans la plage de 1 : 15 à 1 : 1 et préférentiellement dans la plage de 1 : 10 à 1 : 4.

6. Composition selon l'une quelconque des revendications précédentes, dans laquelle le polymère a) possède un poids moléculaire moyen en nombre Mn dans la plage de 5 000 à 9 000 g/mole.

7. Composition selon l'une quelconque des revendications précédentes, dans laquelle le polymère lipophile possède un point de fusion dans la plage de 60 °C à 69 °C et préférentiellement dans la plage de 63 °C à 67 °C.

8. Composition selon l'une quelconque des revendications précédentes, dans laquelle la teneur en polymère a) est de 0,01 % à 10 % en poids, par rapport au poids total de ladite composition.

9. Composition selon l'une quelconque des revendications 1 à 8, dans laquelle l'agent ou les agents écrans d'UV sont choisis parmi des agents écrans d'UV organiques solubles ou insolubles, des agents écrans d'UV minéraux, et des mélanges correspondants.

10. Composition selon l'une quelconque des revendications 1 à 9, dans laquelle la teneur en agents écrans d'UV est de 0,01 % à 60 % en poids, par rapport au poids total de ladite composition.

11. Composition selon l'une quelconque des revendications 1 à 10, dans laquelle lesdits copolymères c) comprennent un ou plusieurs monomères non ioniques choisis parmi des monomères éthyléniquement insaturés solubles dans l'eau, des monomères hydrophobes ou des mélanges correspondants.

12. Composition selon l'une quelconque des revendications précédentes, dans laquelle lesdits copolymères c) comprennent un ou plusieurs monomères non ioniques choisis parmi les monomères solubles dans l'eau non ioniques suivants :
- (méth)acrylamide,
- N-vinylacétamide et N-méthyl-N-vinylacétamide,
- N-vinylformamide et N-méthyl-N-vinylformamide,
- anhydride maléique,
- vinylamine,
- N-vinyllactames comprenant un groupe alkyle cyclique contenant de 4 à 9 atomes de carbone, tel que la N-vinylpyrrolidone, le N-butyrolactame et le N-vinylcaprolactame,
- alcool vinylique de formule CH₂=CHOH,
- monomères vinyliques solubles dans l'eau de formule (B) ci-dessous : 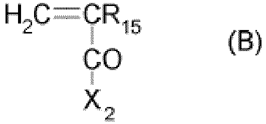 dans laquelle :
- R₁₅ est choisi parmi H, -CH₃, -C₂H₅ et -C₃H₇ ;
- X₂ est choisi parmi :
- des oxydes d'alkyle du type -OR₁₆ dans lesquels R₁₆ est un radical à base d'hydrocarbure saturé ou insaturé, linéaire ou ramifié contenant de 1 à 6 atomes de carbone, éventuellement substitué par un atome d'halogène (iode, brome, chlore ou fluor) ; un groupe hydroxyle (-OH) ; éther.

13. Composition selon l'une quelconque des revendications précédentes, dans laquelle lesdits copolymères c) comprennent un ou plusieurs monomères non ioniques choisis parmi les monomères hydrophobes non ioniques suivants :
- styrène et dérivés correspondants, tels que le 4-butylstyrène, le α-méthylstyrène et un vinyltoluène ;
- acétate de vinyle de formule CH₂=CH-OCOCH₃;
- éthers de vinyle de formule CH₂=CHOR dans laquelle R est un radical à base d'hydrocarbure saturé ou insaturé, linéaire ou ramifié contenant de 1 à 20 et préférablement de 1 à 6 atomes de carbone ;
- acrylonitrile,
- caprolactone ;
- chlorure de vinyle et chlorure de vinylidène ;
- les monomères vinyliques hydrophobes de formule (C) ci-dessous : 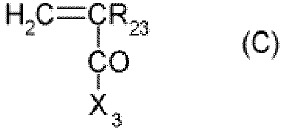 dans laquelle :
- R₃ est choisi parmi H, -CH₃, -C₂H₅ et -C₃H₇, préférablement H,
- X₃ est choisi parmi :
- des oxydes d'alkyle du type -OR₂₄, où R₂₄ est un radical à base d'hydrocarbure saturé ou insaturé, linéaire ou ramifié contenant de 1 à 6 atomes de carbone ;
- les monomères vinyliques de formule (D) ci-dessous : 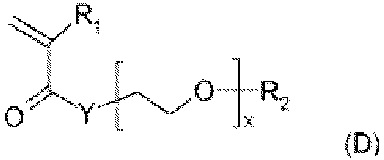 dans laquelle R₁ désigne un atome d'hydrogène ou un radical alkyle en C₁₋₆ linéaire ou ramifié, préférablement méthyle ; Y désigne O ou NH ; R₂ désigne un radical à base d'hydrocarbure hydrophobe comprenant de 8 à 50 atomes de carbone, plus préférentiellement de 8 à 22 atomes de carbone et encore plus préférentiellement de 10 à 18 atomes de carbone ; x désigne un nombre dans la plage de 0 à 100.

14. Composition selon l'une quelconque des revendications précédentes, dans laquelle lesdits copolymères c) sont choisis parmi :
- des copolymères d'acrylamido-2-méthylpropanesulfonate d'ammonium et de vinylpyrrolidone,
- des copolymères de AMPS/méthacrylate de cétéaryle éthoxylés avec 25 moles d'oxyde d'éthylène.

15. Composition selon l'une quelconque des revendications précédentes, dans laquelle lesdits copolymères c) sont présents dans la composition selon l'invention en une quantité dans la plage de 0,1 % à 10 %, préférablement de 0,2 % à 8 % en poids et plus préférentiellement de 0,5 % à 5 % en poids, par rapport au poids total de la composition.

16. Composition selon l'une quelconque des revendications précédentes, **caractérisée en ce qu'**elle comprend également un ou plusieurs agents actifs photosensibles.

17. Composition selon la revendication précédente, dans laquelle l'agent ou les agents actifs photosensibles sont choisis parmi :
- des vitamines et des dérivés correspondants,
- l'acide rétinoïque et des dérivés correspondants, en particulier le rétinol et le palmitate de rétinyle, le benzène-1,4-bis(acide 3-méthylidène-10-camphosulfonique) et des acides n-(C4-C16)acyl-5-salicyliques tels que l'acide n-octanoyl-5-salicylique,
- des polyphénols d'origine naturelle ou synthétique, des dérivés correspondants et des extraits végétaux comprenant ceux-ci,
- et des mélanges correspondants.

18. Composition selon la revendication précédente, dans laquelle lesdits agents actifs photosensibles sont choisis parmi le resvératrol, le rétinol, la baicaline et la vitamine C, et des mélanges correspondants, et préférablement le resvératrol.

19. Composition selon l'une ou l'autre des revendications 17 et 18, dans laquelle l'agent ou les agents actifs photosensibles sont présents en une concentration dans la plage de 0,001 % à 15 %, préférablement de 0,01 % à 10 % et plus particulièrement de 0,1 % à 5 % en poids par rapport au poids total de la composition.
